Europäisches Patentamt

European Patent Office

Office européen des brevets

(11) Veröffentlichungsnummer: **0 309 771 B1**

# (12) EUROPÄISCHE PATENTSCHRIFT

(45) Veröffentlichungstag der Patentschrift: **22.04.92**

(21) Anmeldenummer: **88114414.1**

(22) Anmeldetag: **03.09.88**

(51) Int. Cl.5: **A61M 5/00**

(54) Vorrichtung zum Einspritzen und/oder Entnehmen von Flüssigkeiten.

(30) Priorität: **26.09.87 DE 3732515**

(43) Veröffentlichungstag der Anmeldung:
**05.04.89 Patentblatt 89/14**

(45) Bekanntmachung des Hinweises auf die
Patenterteilung:
**22.04.92 Patentblatt 92/17**

(84) Benannte Vertragsstaaten:
**DE ES FR GB IT SE**

(56) Entgegenhaltungen:
**US-A- 4 512 766**

(73) Patentinhaber: **JOKA KATHETERTECHNIK
GMBH
Im Weiher 19
W-7450 Hechingen(DE)**

(72) Erfinder: **Sunnanväder, Lars
Ermelesstrasse 37
W-7450 Hechingen(DE)**
Erfinder: **Hartig, Thomas
Zu den Linden 14
W-7450 Hechingen-Bechtoldsweiler(DE)**

(74) Vertreter: **Weber, Dieter, Dr. et al
Dr. Dieter Weber und Dipl.-Phys. Klaus Seiffert Patentanwälte Gustav-Freytag-Strasse
25 Postfach 6145
W-6200 Wiesbaden 1(DE)**

**Beschreibung**

Die Erfindung betrifft eine Vorrichtung zum Einspritzen und/oder Entnehmen von Flüssigkeiten in oder aus einem Kanal, insbesondere Körperflüssigkeiten im Kanal eines Kanülenanschlußstückes, mit einem sich an das Kanalgehäuse anschließenden Führungszylinder, in dem ein mit dem Kanal in Fließverbindung stehendes, als Rohr ausgebildetes Verbindungsteil angeordnet ist, dessen freies Ende durch ein Elastomerstück abdichtbar ist und dessen freies Ende von außen von dem Elastomerstück kappenartig überdeckt und abgedichtet ist, wobei das Elastomerstück an dem als Ventilgehäuse ausgestalteten Führungszylinder gehalten ist, wobei an dem äußeren freien Ende des Führungszylinders eine ein freies Ende einer Spritze oder dergleichen zum Einspritzen oder Entnehmen von Flüssigkeit aufnehmende Aufnahmeeinrichtung ausgebildet ist, deren konzentrisch zum Rohrverlaufende Wandung mit der Außenfläche des Rohres einen Ringraum bildet, der für die Aufnahme des freien Endes der Spritze oder dergleichen ausreichend groß ist und wobei beim Einsetzen des freien Endes der Spritze oder dergleichen in die Aufnahmeeinrichtung das freie Ende des Elastomerstückes relativ zu dem Rohr derart verschoben wird, daß es von dem freien Ende des Rohres durchstochen wird und dabei eine Flüssigkeitsverbindung von der Spritze oder dergleichen zum Kanal herstellt.

Derartige Einspritzvorrichtungen werden in der Praxis derzeit in Krankenhäusern benutzt. Hier ist der Kanülenkopf über eine Verbindungsöffnung radial mit einem Zylinder verbunden, dessen Inneres mit dem Kanal im Kanülenkopf in Fließverbindung steht. Im Bereich dieser seitlich an einer Stelle im Kanülenkopf befindlichen Verbindungsöffnung liegt ein Elastomerstück in Form eines Gummischlauches. Hierdurch ist eine Art Rückschlagventil derart gebildet, daß nach dem Einstechen der Stahlkanüle bzw. Verbinden des Katheterrohres mit dem betreffenden Blutgefäß Blut durch das am Innenumfang des Kanülenkopfes anliegende Schlauchstück hindurchfließen bzw. hinten abgenommen werden kann. Über den Führungszylinder kann man von oben Schläuche anstecken und in Richtung entgegen der Fließrichtung des Blutes Flüssigkeiten in das Blutgefäß einführen, z.B. bei den Infusionen.

Der Nachteil dieser bekannten Einführvorrichtung mit Kathetern und Infusionssystemen besteht einerseits in der Gefahr der Kontamination, weil nämlich nach Abnehmen der Schutzkappe von dem Führungszylinder durch Berührung oder dergleichen Verunreinigungen an die Kappe einerseits und über die Außenwandungen der Spritze vorn andererseits eingeführt und mittels der Flüssigkeiten dann in das Blutsystem eines Patienten eingeschleppt werden. Spätestens nach Aufsetzen der verunreinigten Abdeckkappe werden Bazillen in die Innenwandungen des Führungszylinders gedrückt und bei der nächsten Benutzung in das Blutgefäß mit hineingerissen. Selbstverständlich ist es unerwünscht, wenn eine solche Vorrichtung die Infektionsträger in das Blutsystem eines Patienten durchläßt. Nachteilig ist es bei dieser bekannten Vorrichtung aber auch, daß das Elastomerstück eine Art Rückschlagventil bildet und nicht die Entnahme von Körperflüssigkeit erlaubt. Ungünstig ist ferner die Reinigungsmöglichkeit der gesamten Vorrichtung des bekannten Kanülenanschlußstükkes.

Bekannt und mit Nachteilen behaftet ist auch eine in USA unter anderem angewandte Methode bei Injektionen in Infusionssystemen mit dem Punktieren eines Latexschlauches mittels einer Stahlnadel, wobei ausgestanzte Teilchen in das Blutsystem gelangen können.

Weiterhin ist aus der US-A-4,512,766 eine Einspritzvorrichtung bekannt, bei der sich an den im Kanalgehäuse ausgebildeten Kanal ein Rohr anschließt, welches sich in den einteilig mit dem Kanalgehäuse ausgebildeten Führungszylinder erstreckt und welches ebenfalls im Abschnitt einer am Führungszylinder angeordneten, als Bohrung ausgebildeten Aufnahmeeinrichtung endet, die zur Aufnahme eines an einem Abgabe- oder Entnahmebehälter, beispielsweise einer Spritze, angeordneten freien Endes dient. Das freie Ende des Rohres ist durch ein Elastomerstück dann abgedichtet, wenn die Spritze oder dergleichen mit ihrem freien Ende nicht in die Aufnahmeeinrichtung eingeführt bzw. nicht ihre ordnungsgemäße Endstellung in der Aufnahmeeinrichtung erreicht hat.

Um bei dieser Einspritzeinrichtung eine Flüssigkeitsverbindung zwischen der Kammer der Spritze und dem Rohr herzustellen, muß das freie Ende der Spritze soweit in die Aufnahmebohrung eingeführt werden, bis durch axiale Verschiebung des freien Endes des Elastomerstückes durch das freie Ende der Spritze der Durchbruch von dem Rohr durchstochen wird. Bei dieser Einspritzeinrichtung ist es als weniger vorteilhaft anzusehen, daß der als Klappenventil ausgebildete Durchbruch leicht verschmutzen kann und daß nach mehrmaligem Öffnen des Durchbruchs durch das Rohr dieser nicht mehr einwandfrei abdichtet.

Aufgabe der Erfindung ist daher die Verbesserung einer Vorrichtung der eingangs genannten Art dahingehend, daß die beschriebene Kontamination vermieden wird und daß nach dem Zurückziehen der Abgabe- oder Entnahmeeinrichtung die vom Rohr im Elastomerstück gebildete Einstichstelle wieder zuverlässig umschlossen wird.

Erfindungsgemäß wird diese Aufgabe dadurch gelöst, daß das Elastomerstück in seinem radial äußeren Bereich und im Bereich des freien Endes des Rohres von einer Spannhülse unter radial zur Mitte hin wirkendem Druck zusammengepreßt ist. Der große Vorteil dieser Spannhülse besteht darin, daß ein eingestochenes Loch im Elastomerstück nach dem ersten Benutzen garantiert nach dem Zurückziehen der Spritze wieder verschlossen wird. Durch das Zusammenpressen des Elastomerstükkes mit Hilfe der Spannhülse, die gleichzeitig auch ein Hochwölben des Elastomerstückes am äußeren freien Ende bewirkt, läßt sich die Einstichstelle stets wieder dicht verschließen, so daß vor der jeweiligen Benutzung das Pflegepersonal die Einstichstelle sehr leicht und gründlich abwischen und damit dekontaminieren kann, was eine leichte Reinigung der Einstichstelle von außen ermöglicht.

Besonders zweckmäßig ist es, daß das Elastomerstück das Rohr bis in die Nähe dessen Befestigungsbereiches becherförmig umgreift und daß das Elastomerstück mit seinem dem freien Ende gegenüberliegenden Ende am inneren Ende des Führungszylinders befestigt ist.

Ein weiterer erheblicher Vorteil dieser Ausführungsform besteht darin, daß die Aufnahmeeinrichtung am äußeren freien Ende des Führungszylinders ausgebildet ist und daß die Aufnahmeeinrichtung von einem Luer-Aufnahmekonus gebildet wird, in den das ebenfalls als Luer-Einsteckkonus ausgebildete freie Ende beim Aufsetzen der Spritze oder dergleichen dichtend eingreift. Die Spritzen sind bekanntlich normiert und haben einen Luer-Einsteckkonus, so daß es sehr vorteilhaft ist, wenn die erfindungsgemäße Vorrichtung einen solchen Luer-Einsteckkonus aufnehmen kann. Die Spritze ist also nach dem Einstecken in den Führungszylinder dort fest abgestützt, d.h., die Position einer eingesteckten Spritze läßt sich durch diese Maßnahmen besonders gut fixieren. Würde nämlich das vordere Ende der Spritze zwischen Führungszylinder und Rohr lediglich so eingesteckt werden, daß der vordere Luer-Einsteckkonus der Spritze nur über das Elastomerstück gegen die Innenfläche des Führungszylinders zur Anlage käme, dann wäre die Spritze im Führungszylinder nicht gut fixiert oder gehalten. Das häufig aus Latex bestehende, membranartige Elastomerstück ist in aller Regel so weich, daß man die Spritze nach der Benutzung ohne die erfindungsgemäß vorgesehenen Fixiermaßnahmen nicht stecken lassen könnte, weil sie mangels Halterung sonst umfallen würde.

Nun ist es ferner aber von besonderem Vorteil, wenn das Elastomerstück im Mittelbereich des Rohres dünnere Wandungen als im Bereich des freien Endes des Rohres aufweist und dadurch zwischen Rohr und Führungszylinder mindestens ein freier Ringraum gebildet wird. Das Anstecken oder Abziehen von Spritzen soll möglichst leichtgängig und ohne Kraftaufwendungen möglich sein, um die Behandlungen des Patienten schmerzfrei und angenehm zu gestalten. Zwar ist einerseits die Dichtigkeit eine zwingende Voraussetzung für die Funktion der erfindungsgemäßen Vorrichtung, andererseits wird aber durch die vorstehenden Maßnahmen erreicht, daß gegenüber dem Rohr als Verbindungsstück eine geringere Reibung geschaffen wird, wozu der genannte Ringraum nützlich ist. Andererseits ist der Ringraum groß genug ausgestaltet - oder es sind mehrere Ringräume nebeneinander angeordnet -, so daß beim Einstecken des Luer-Einsteckkonus das Elastomerstück mit seiner Masse in vorhandene Räume ausweichen kann, so daß dem Zusammendrücken des Elastomerstückes nicht ein zu großer Widerstand entgegengesetzt wird. Durch die Ringräume ist also Platz genug vorhanden, wo das Elastomerstück bei der Zusammenpressung hinbewegt werden kann.

Zweckmäßig ist die Erfindung weiterhin dadurch ausgestaltet, daß die Spannhülse am inneren Ende einen radial nach außen ragenden Flansch zur Abstützung einerseits gegen eine entsprechende Schulter des Elastomerstückes sowie andererseits gegen eine Schulter im Führungszylinder aufweist. Auf diese Weise kann man mit dem freien Ende des Luer-Einsteckkonus einfach auf diese Spannhülse drücken, durch welche das Elastomerstück mitgenommen und über die erwähnte Schulter bewegbar und gleichzeitig auch günstig verformbar ist. Dabei ist es von Vorteil, wenn ein oder mehrere Ringräume neben der Schulter des Elastomerstückes angeordnet sind. Die Spannhülse zieht das Elastomerstück im Bereich der Spannhülse derart auf die Mitte bzw. das Zentrum des Elastomerstückes zusammen, daß eine hohe Vorspannung gegeben ist, die stets ein Schließen des einmal eingestochenen Loches garantiert. Dadurch erfolgt auch die Verformung hauptsächlich außerhalb des Bereiches der Spannhülse, so daß das Elastomerstück innerhalb der Spannhülse bis auf das Öffnen und Schließen der Durchstechöffnung unverformt bleibt. Im wesentlichen besteht mit weiterem Vorteil die einzige Reibung beim Ansetzen der Spritze bis zum Einführen oder Abziehen von Flüssigkeiten in der Gleitberührung zwischen dem Elastomerkörper und dem Verbindungsrohr, und hier vorzugsweise sogar lediglich im oberen, äußeren Bereich der Spannhülse, etwa in deren oberer Hälfte, so daß der Widerstand beim Einstecken des Luer-Einsteckkonus ersichtlich gering ist.

Die Erfindung ist ferner weiter vorteilhaft dadurch ausgestaltet, daß die Schulter im Führungszylinder das innere Ende des Luer-Aufnahmekonus des Führungszylinders ist. Der Luer-Einsteckkonus kann dadurch also in eine definierte Tiefe eingesteckt werden, so daß das Elastomerstück einen definierten Hub und damit lediglich die vorbestimmte Deformation erleidet. Die Füllung des vorhandenen Raumes ist also genau gesteuert, wobei alle diese Teile einfach herstellbar und leicht zu montieren sind, so daß die erfindungsgemäße Vorrichtung insgesamt preiswert herstellbar und gut verkäuflich ist, weil das Pflegepersonal die angenehme Benutzung zu schätzen lernt.

Günstig ist es erfindungsgemäß auch, wenn das Rohr aus Kunststoff besteht und einstückig mit dem Kanalgehäuse geformt ist. Bei einer ersten Ausführungsform kann man ein Metallrohr in eine Öffnung im Kanalgehäuse unter Einpressen einführen und dort befestigen. Das Kanalgehäuse hat im allgemeinen aber eine ausreichende Härte, so daß man aus demselben Material zugleich das Rohr anformen kann, wie vorstehend empfohlen. Dabei ist ein besonderer Arbeitsgang zum Einsetzen des Metallrohres überflüssig, wodurch sich die gesamte Vorrichtung noch weiter verbilligt. Am freien Ende ist auch dieses Kunststoffrohr scharf genug, so daß das vorgespannte Elastomerstück einwandfrei beim Einstecken des Luer-Einsteckkonus durchstochen werden kann. Es ist zweckmäßig, wenn man dieses aus Kunststoff bestehende Verbindungsrohr etwas konisch gestaltet derart, daß es sich zum oberen freien Ende hin verjüngt.

Weiterhin ist es erfindungsgemäß vorteilhaft, wenn der Führungszylinder an seinem äußeren freien Ende durch eine angelenkte Abdeckkappe verschließbar ist und an seinem inneren Befestigungsende mittels einer Schnappeinrichtung am Kanalgehäuse angebracht ist unter axialer Einpressung eines Radialflansches des Elastomerstückes. Als Schnappeinrichtung kann beispielsweise radial eine Wulst außen am unteren inneren Ende des Führungszylinders vorstehen und in eine entsprechende innere Nut im Kanalgehäuse eingreifend verwendet werden. Ferner kann in der Nachbarschaft dieser Schnappeinrichtung ein nach außen radial vorstehender Radialflansch des Elastomerstückes eine axiale Pressung (Achsrichtung des Rohres) derart erfahren, daß eine einwandfreie Ringdichtung gegeben ist, die einerseits eine zuverlässige Abdichtung zwischen dem das Rohr umgebenden Ringraum nach außen sicherstellt und andererseits eine zuverlässige Befestigung des Elastomerstückes innerhalb des Führungszylinders vorsieht. Am äußersten inneren Ende ist das Elastomerstück also im Führungszylinder befestigt und ist in seinen anderen Berei chen bis zum oberen freien Ende frei beweglich. Hierdurch sind die oben beschriebenen Bewegungen, Deformationen und Massenverschiebungen besonders gut möglich.

Mit Hilfe der erwähnten Abdeckkappe läßt sich die Verunreinigungsgefahr weiterhin verringern. Das Anformen einer Abdeckkappe am Führungszylinder ist mit der heutigen Spritztechnik leicht möglich.

Weiterhin ist die Erfindung vorteilhaft dadurch ausgestaltet, daß das Elastomerstück aus Gummi, Latex oder einem Latex-Silicongemisch besteht. Diese Materialien haben sich als zuverlässig, mechanisch und chemisch einwandfrei erwiesen und zeigen auch eine lange Standzeit.

Weitere Vorteile, Merkmale und Anwendungsmöglichkeiten der vorliegenden Erfindung ergeben sich aus der folgenden Beschreibung bevorzugter Ausführungsbeispiele anhand der folgenden Beschreibung. Es zeigen:

Fig. 1    die Schnittansicht einer erfindungsgemäßen Vorrichtung mit unten abgebrochenem Kanalgehäuse, in geschlossenem, unbenutztem Zustand,

Fig. 2    die gleiche Schnittdarstellung, bei der jedoch eine Spritze eingesetzt ist, die Abdeckkappe beiseitegeklappt ist und das Elastomerstück zusammengepreßt ist; und

Fig. 3    eine Querschnittsansicht wie Figur 1, jedoch bei einer anderen Ausführungsform, bei welcher das Verbindungsteil nicht ein Metallrohr sondern ein Rohr aus Kunststoff ist, welches einstückig mit dem Kanalgehäuse geformt wurde.

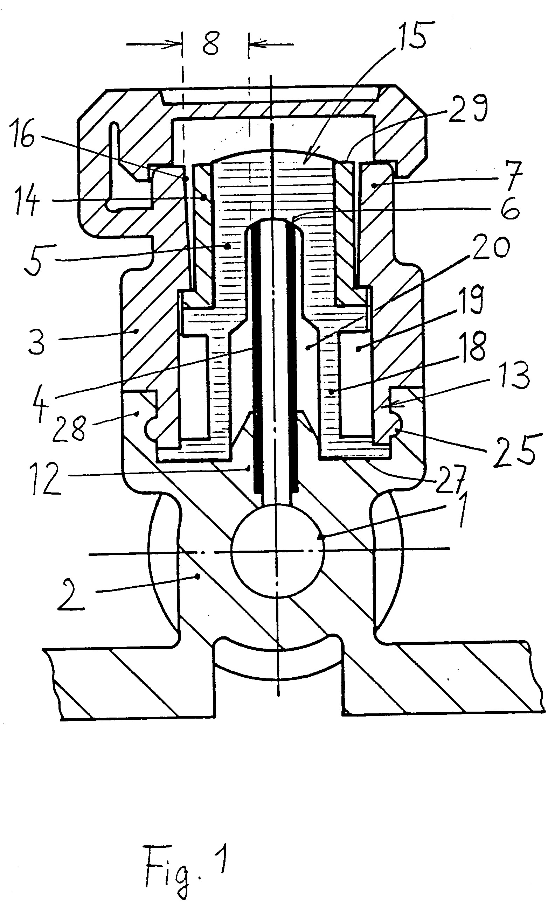

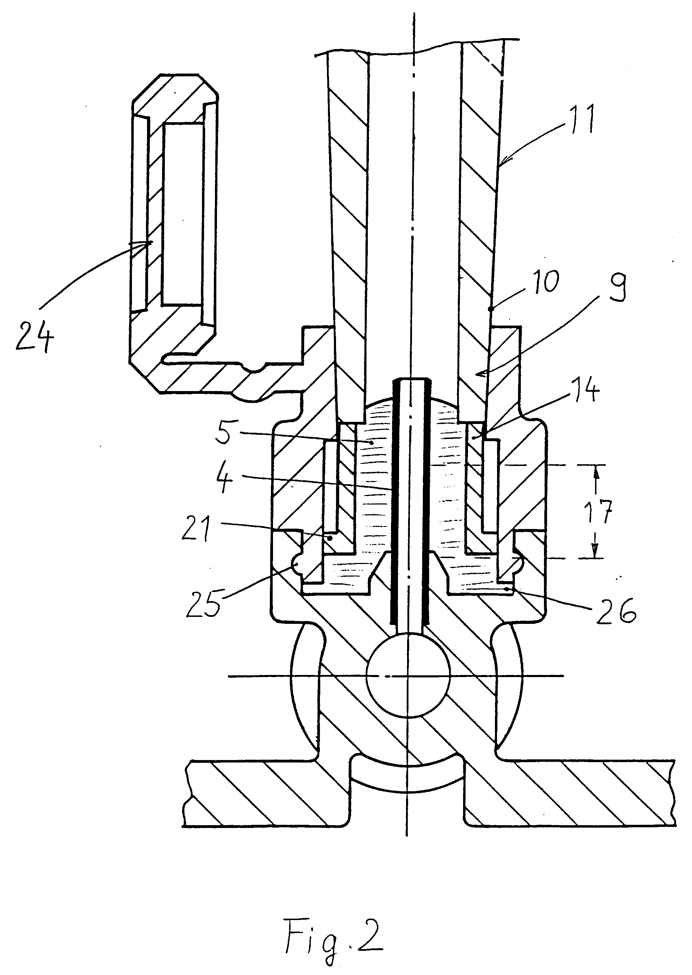

Im Kanal 1 innerhalb des Kanalgehäuses, welches mit dem abgebrochen gezeigten Kanülenkopf in Verbindung steht, ist der Durchlaß für Blut oder eine andere Körperflüssigkeit gegeben. Diese Kanal 1 hat nach oben in der Mitte eine Verbindungsöffnung zu dem Verbindungsstück 4 hin, welches in der Ausführungsform der Fig. 1 und 2 ein Metallrohr ist. Man kann dieses auch als Kanüle bezeichnen. Dieses Metallrohr ist in seinem Befestigungsbereich bei der Herstellung unter Druck in eine Öffnung im Kanalgehäuse 2 eingepreßt und sitzt deshalb starr und fest.

Das Kanalgehäuse erweitert sich außerhalb des Kanals 1 im unteren inneren Bereich des Verbindungsrohres 4 becherartig und weist eine innere Ringnut auf, welche zu der Schnappeinrichtung 25 gehört. Dieses obere becherartige Ende des Kanalgehäuses 2 weist eine ebene Ringfläche 27 auf, die im Abstand von dem Verbindungsrohr 4 innen beginnt und an der hochstehenden Ringwandung 28 dieses becherförmigen Teils endet.

Ein Führungszylinder 3, der am oberen Ende eine Abdeckkappe 24 angeformt aufweist, greift an seinem inneren unteren Ende 13 in diese Ringwand 28, d.h. in das becherförmige Ende des Kanalgehäuses 2, ein, wobei eine radial nach außen vorstehende Ringwulst, die zu der Schnappeinrichtung 25 gehört, so vorsteht und bemessen ist, daß beim Eindrücken des Führungszylinders 3 von oben auf das Kanalgehäuse 2 die Ringwulst in die Ringnut einschnappt, wodurch die Schnappeinrichtung 25 eine feste Verbindung zwischen dem Führungszylinder 3 und dem Kanalgehäuse 2 schafft.

Ein Elastomerstück 5 aus Latex oder einem Gemisch von Latex und Silicon ist wie ein Kolben ausgestaltet und übergreift das freie Ende 6 des Verbindungsrohres 5 abdichtend. An dieser Abdichtstelle ist das Elastomerstück 5 vorpunktiert, so daß bei einer Relativbewegung zwischen dem Elastomerstück 5 und dem Verbindungsrohr 4 ein Durchstechen des Elastomerstückes 5 erfolgt, wie im Zustand der Fig. 2 nach dem Durchstechen dargestellt ist.

Das Elastomerstück 5 ist in seinem oberen Bereich, etwa in seiner oberen Hälfte, von einer Spannhülse 14 unter Spannung so umgeben, daß sich der äußere Bereich 15 des Elastomer stückes 5 nach außen wölbt, wie in allen drei Fig. dargestellt ist.

Die Spannhülse 14 ist außen etwa zylinderförmig und liegt im oberen Teil des Führungszylinders 3, während die der Spannhülse 14 gegenüberliegende Innenfläche des Führungszylinders 3 als Luer-Aufnahmekonus 16 ausgestaltet ist.

Der Abstand zwischen dieser kegelstumpfförmig ausgestalteten Ringfläche des Luer-Aufnahmekonus 16 einerseits und der zylindermantelförmigen Außenfläche des Verbindungsrohrs 4 andererseits ist mit 8 bezeichnet und wird der Klarheit wegen Ringabstand genannt. Dieser Ringabstand 8 ist so bemessen, daß das vordere freie Ende 9 des Luer-Einsteckkonus 10 einer Spritze 11 gut aufgenommen werden kann, wie man in Figur 2 sieht.

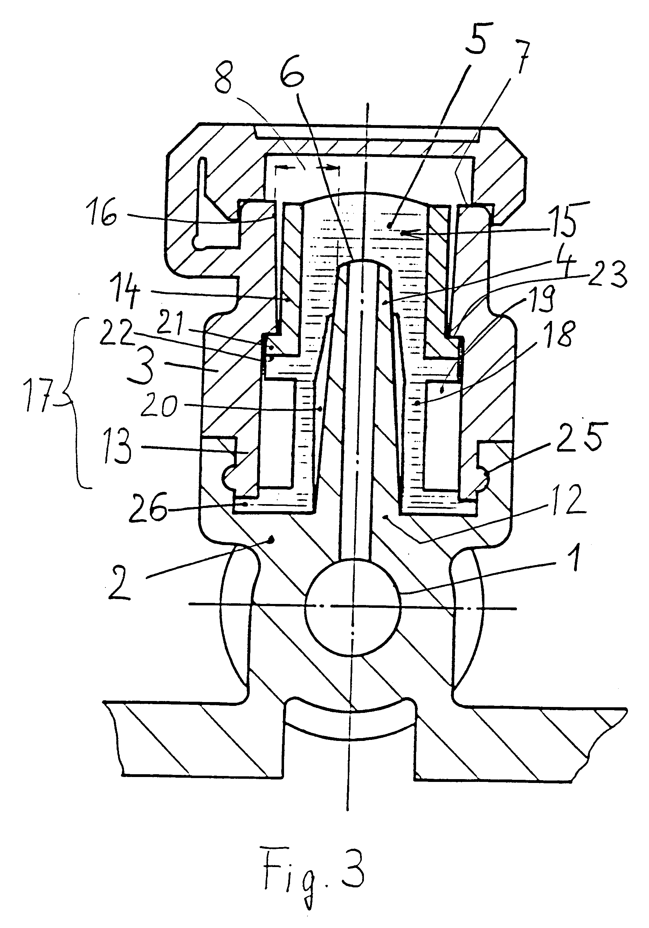

In dem in den Figuren 2 und 3 gezeigten Mittelbereich 17 des Verbindungsrohres 4 hat das Elastomerstück 5 dünnere Wandungen 18 als im Bereich des freien Endes 6 des Rohres 4. Dadurch sind zwei freie Ringräume 19, 20 zwischen dem Rohr 4 einerseits und dem Führungszylinder 3 andererseits gebildet. Es handelt sich hierbei zum einen um den außerhalb des Elastomerstückes 5 und außerhalb der Wandung 18 desselben angeordneten äußeren Ringraum 19; und den innerhalb der Wandung 18 aber außerhalb des Verbindungsrohres 4 befindlichen inneren Ringraum 20. Man sieht, daß der innere Ringraum 20 nach unten vom Befestigungsbereich 12 des Kanalgehäuses 2 begrenzt ist, während sich nach außen oben ein verengter Ringraum anschließt, welcher das gesamte Verbindungsrohr 4 umgibt.

Ähnlich verhält es sich bei der Ausführungsform der Figur 3, nur daß der äußere Ringraum 20 das angeformte Kunststoff-Verbindungsrohr 4 im wesentlichen umgreift. Wegen des weniger festen Materials Kunststoff als Metall ist dieser äußere Ringraum 20 bei der zweiten Ausführungsform nach Figur 3 kleiner gehalten.

Das insgesamt im Querschnitt U-förmig oder auch becherförmig ausgestaltete Elastomerstück weist durch die erfindungsgemäße Ausgestaltung in seinem Mittelbereich und auch an seinem freien Ende, welches der untere innere Bereich ist, einen radial nach außen ragenden Flansch auf, von denen der untere innere als Radialflansch 26 bezeichnet ist. Dieser wird am inneren Ende 13 des Führungszylinders 3 unter Druck eingequetscht und durch die Schnappeinrichtung 25 in dieser zusammengepreßten Position gehalten, wie die Figuren zeigen, so daß das Elastomerstück 5 an dieser Stelle einwandfrei fest gehalten ist.

Der im mittleren Bereich befindliche Radialflansch bildet die Schulter 22 des Elastomerkörpers 5, welche dem radial vorstehenden Flansch 21 der Spannhülse 14 gegenüberliegt. Drückt man also von oben mit der Spritze 11 nach unten auf die Spannhülse 14, so wird über den Flansch 21 und die Schulter 22 des Elastomerkörpers 5 die Deformation des unteren Bereiches des Elastomerstückes 5, nämlich das Verquetschen der Wandungen 18, eingeleitet.

Am unteren inneren Ende des Luer-Aufnahmekonus 16 des Führungszylinders 3 ist eine weitere Schulter 23 gebildet, welche wiederum mit dem Flansch 21 der Spannhülse 14 zusammenwirkt. Über diese Schulter/Flanschverbindung ist nämlich die Spannhülse 14 unverlierbar im Führungszylinder 3 gehalten.

Man erkennt den kegelstumpfförmigen Ringraum außerhalb der Spannhülse 14 und innerhalb des Luer-Aufnahmekonus 16; mit dem Vorteil, daß beim Herunterdrücken der Spannhülse 14 vom Zustand der Figuren 1 und 3 in den Zustand der Figur 2 lediglich die Ringkante an der Schulter 23 des Führungszylinders 3 mit der Spannhülse 14 in Linienberührung kommt. Durch diese geringe Berührung ergibt sich auch eine geringe Reibung und damit ein geringer Widerstand gegenüber den Benutzungskräften.

Im Betrieb der Ausführungsform nach den Figuren 1 und 2 wird zunächst die Vorrichtung im Zustand der Figur 1 angetroffen. Das Pflegepersonal öffnet die Abdeckkappe 24 und setzt das vordere freie Ende 9 des Luer-Einsteckkonus 10 der Spritze 11 auf die obere freie Ringfläche 29 der Spannhülse 14 auf und drückt den Luer-Einsteckkonus 10 nach unten in Richtung auf den Kanal 1. Diese Bewegung ist durch den Ringabstand 8 möglich. An der vorpunktierten Stelle am freien Ende 6 des Verbindungsrohres 4 wird das Elastomerstück 5 in seinem äußeren Bereich 15 eingestochen, durchstochen und über das Verbindungsrohr 4 nach unten geschoben, bis der Zustand der Figur 2 erreicht ist. Dies ist dadurch der Endzustand, daß die äußere Fläche des Luer-Einsteckkonus 10 mit der Fläche des Luer-Aufnahmekonus 16 in vollen Anschlag kommt. Eine weitere Bewegung der Spritze 11 auf den Kanal 1 zu ist deshalb nicht möglich. Das Verbindungsrohr 4 ragt nun ein Stück weit aus dem Elastomerstück 5 nach außen heraus, so daß zwischen dem Inneren der Spritze 11 und dem Kanal 1 eine sichere und offene Fließverbindung für Flüssigkeiten gegeben ist.

Beim Hereindrücken wird das Material des Elastomerstückes 5 insbesondere im unteren Bereich zusammengepreßt, wobei sich der zur Verfügung stehende freie Raum in Gestalt der freien Ringräume 19 und 20 laufend verkleinert. Dies geschieht durch das Ausdehnen des von oben nach unten drängenden Materials des Elastomerstückes 5. Schließlich sind im Zustand der Figur 2 alle freien Räume ausgefüllt, und es sind zusätzlich zu der schon vorhandenen Dichtung im Bereich des Radialflansches 26 einwandfreie Dichtungen gegeben. Die Spritze 11 sitzt fest abgestützt im Luer-Aufnahmekonus 16, so daß ein Umkippen nicht befürchtet werden muß.

Wenn nach Einspritzen oder Entnehmen der Flüssigkeit die Spritze 11 abgenommen wird, sorgt die Vorspannkraft des Elastomerstückes 5 dafür, daß dieses sich vom Zustand der Figur 2 in den der Figur 1 zurückbewegt. Dabei wird die Spannhülse 14 wieder nach außen oben axial derart verschoben, bis der Flansch 21 gegen die Schulter 23 zur Anlage kommt und die Wandungen 18 wieder ihre ursprüngliche Gestalt erreichen.

Durch die Vorspannung des Elastomerstückes 15 in seinem äußeren Bereich 15 durch die Spannhülse 14 wird das in Figur 2 gezeigte Loch im Elastomerstück garantiert wieder verschlossen.

**Patentansprüche**

1. Vorrichtung zum Einspritzen und/oder Entnehmen von Flüssigkeiten in oder aus einem Kanal (1), insbesondere Körperflüssigkeiten im Kanal eines Kanülenanschlußstückes, mit einem sich an das Kanalgehäuse (2) anschließenden Führungszylinder (3), in dem ein mit dem Kanal (1) in Fließverbindung stehendes, als Rohr ausgebildetes Verbindungsteil (4) angeordnet ist, dessen freies Ende (6) durch ein Elastomerstück (5) abdichtbar ist und dessen freies Ende (6) von außen von dem Elastomerstück (5) kappenartig überdeckt und abgedichtet ist, wobei das Elastomerstück (5) an dem als Ventilgehäuse ausgestalteten Führungszylinder (3) gehalten ist, wobei an dem äußeren freien Ende (7) des Führungszylinders (3) eine ein freies Ende (9) einer Spritze (11) oder dergleichen zum Einspritzen oder Entnehmen von Flüssigkeit aufnehmende Aufnahmeeinrichtung (16) ausgebildet ist, deren konzentrisch zum Rohr (4) verlaufende Wandung mit der Außenfläche des Rohres (4) einen Ringraum (8) bildet, der für die Aufnahme des freien Endes (9) der Spritze (11) oder dergleichen ausreichend groß ist und wobei beim Einsetzen des freien Endes (9) der Spritze (11) oder dergleichen in die Aufnahmeeinrichtung (16) das freie Ende des Elastomerstückes (5) relativ zu dem Rohr (4) derart verschoben wird, daß es von dem freien Ende (6) des Rohres (4) durchstochen wird und dabei eine Flüssigkeitsverbindung von der Spritze (11) oder dergleichen zum Kanal (1) herstellt, dadurch gekennzeichnet, daß das Elastomerstück (5) in seinem radial äußeren Bereich (15) und im Bereich des freien Endes (6) des Rohres (4) von einer Spannhülse (14) unter radial zur Mitte hin wirkendem Druck zusammengepreßt ist.

2. Vorrichtung nach Anspruch 1, dadurch gekennzeichnet, daß das Elastomerstück (5) das Rohr (4) bis in die Nähe dessen Befestigungsbereiches (12) becherförmig umgreift und daß das Elastomerstück (5) mit seinem dem freien Ende gegenüberliegenden Ende am inneren Ende (13) des Führungszylinders (3) befestigt ist.

3. Vorrichtung nach Anspruch 1, dadurch gekennzeichnet, daß die Aufnahmeeinrichtung (16) am äußeren freien Ende (7) des Führungszylinders (3) ausgebildet ist und daß die Aufnahmeeinrichtung (16) von einem Luer-Aufnahmekonus (16) gebildet wird, in den das ebenfalls als Luer-Einsteckkonus ausgebildete freie Ende (9) beim Aufsetzen der Spritze (11) oder dergleichen dichtend eingreift.

4. Vorrichtung nach einem der Ansprüche 1 oder 2, dadurch gekennzeichnet, daß das Elastomerstück (5) im Mittelbereich (17) des Rohres (4) dünnere Wandungen (18) als im Bereich des freien Endes (6) des Rohres (4) aufweist und dadurch zwischen Rohr (4) und Führungszylinder (3) mindestens ein freier Ringraum (19, 20) gebildet ist.

5. Vorrichtung nach einem der Ansprüche 1 bis 4, dadurch gekennzeichnet, daß die Spannhülse (14) am inneren Ende einen radial nach außen ragenden Flansch (21) zur Abstützung einerseits gegen eine entsprechende Schulter (22) des Elastomerstückes (5) sowie andererseits gegen eine Schulter (23) im Führungszylinder (3) aufweist.

6. Vorrichtung nach Anspruch 3 und 5, dadurch gekennzeichnet, daß die Schulter (23) im Führungszylinder (3) das innere Ende des Luer-Aufnahmekonus (16) des Führungszylinders (3) ist.

7. Vorrichtung nach einem der Ansprüche 1, 2 oder 4, dadurch gekennzeichnet, daß das Rohr (4) aus Kunststoff besteht und einstückig mit dem Kanalgehäuse (2) geformt ist.

8. Vorrichtung nach Anspruch 1, dadurch gekennzeichnet, daß der Führungszylinder (3) an seinem äußeren freien Ende (7) durch eine angelenkte Abdeckkappe (24) verschließbar ist und an seinem inneren Befestigungsende mittels einer Schnappeinrichtung (25) am Kanalgehäuse (2) angebracht ist unter axialer Einpressung eines Radialflansches (26) des Elastomerstückes (5).

9. Vorrichtung nach einem der Ansprüche 1, 2, 4 oder 5, dadurch gekennzeichnet, daß das Elastomerstück (5) aus Gummi, Latex oder einem Latex-Silikongemisch besteht.

**Claims**

1. A device for injecting and/or withdrawing fluids into or out of a duct (1), in particular body fluids in the duct of a cannula connecting portion, having a guide cylinder (3) which is connected to the duct housing (2) and in which is disposed a connecting part (4) which is in flow communication with the duct (1) and which is in the form of a tube and whose free end (6) can be sealed off by an elastomer portion (5) and whose free end (6) is covered over cap-like fro the outside by the elastomer portion (5) and is sealed off, wherein the elastomer portion (5) is held to the guide cylinder (3) which is in the form of a valve housing, wherein provided at the outer free end (7) of the guide cylinder (3) is a receiving means (16) which receives a free end (9) of a syringe (11) or the like for the injection or removal of fluid and whose wall, which extends concentrically with respect to the tube (4), forms with the outside surface of the tube (4) an annular space (8) which is sufficiently large to receive the free end (9) of the syringe (11) or the like, and wherein upon insertion of the free end (9) of the syringe (11) or the like into the receiving means (16), the free end of the elastomer portion (5) is displaced relative to the tube (4) in such a way that it is pierced by the free end (6) of the tube (4) and thus makes a fluid communication from the syringe (11) or the like to the duct (1), characterised in that in its radially outward region (15) and in the region of the free end (6) of the tube (4) the elastomer portion (5) is compressed by a clamping sleeve (14) under a pressure which acts radially towards the centre.

2. A device according to claim 1 characterised in that the elastomer portion (5) surrounds the tube (4) in a cup-like configuration to a position in the vicinity of the fixing region (12) thereof and that the elastomer portion (5) is fixed to the inner end (13) of the guide cylinder (3), by its end in opposite relationship to the free end.

3. A device according to claim 1 characterised in that the receiving means (16) is provided at the outer free end (7) of the guide cylinder (3) and that the receiving means (16) is formed by a luer receiving cone (16) into which the free end (9), which is also in the form of a luer insertion cone, sealingly engages when the syringe (11) or the like is fitted.

4. A device according to one of claims 1 and 2 characterised in that in the middle region (17) of the tube (4) the elastomer portion (5) has thinner walls than in the region of the free end (6) of the tube (4) and in that way at least one free annular space (19, 20) is formed between the tube (4) and the guide cylinder (3).

5. A device according to one of claims 1 to 4 characterised in that at the inner end the clamping sleeve (14) has a radially outwardly projecting flange (21) for bearing on the one hand against a corresponding shoulder (22) on the elastomer portion (5) and on the other hand against a shoulder (23) in the guide cylinder (3).

6. A device according to claims 3 and 5 characterised in that the shoulder (23) in the guide cylinder (3) is the inner end of the luer receiving cone (16) of the guide cylinder (3).

7. A device according to one of claims 1, 2 and 4 characterised in that the tube (4) comprises plastics meterial and is formed integrally with the duct housing (2).

8. A device according to claim 1 characterised in that the guide cylinder (3) is closable at its outer free end (7) by a pivotally mounted cover cap (24) and at its inner fixing end is mounted to the duct housing (2) by means of a snap device (25), with the radial flange (26) of the elastomer portion (5) being axially pressed in.

9. A device according to one of claims 1, 2, 4 and 5 characterised in that the elastomer portion (5) comprises rubber, latex, or a latex-rubber blend.

**Revendications**

1. Dispositif destiné à injecter des liquides dans un canal (1) et/ou à les en prélever, notamment des fluides corporels dans le canal d'une pièce de raccordement de canule, comportant un cylindre de guidage (3), lui-même raccordé à l'enveloppe de canal (2), et dans lequel est disposé un adaptateur (4), en relation d'écoulement avec le canal (1) et constitué d'un tube, adaptateur dont l'extrémité libre (6) peut être rendue étanche grâce à une pièce en élastomère (5), et dont l'extrémité libre (6) est recouverte et étanchée de l'extérieur, comme par un capuchon, par la pièce en élastomère (5), la pièce en élastomère (5) étant maintenue contre le cylindre de guidage (3), lequel est conçu comme une cage de soupape ; à l'autre extrémité libre (7) du cylindre de guidage (3) se trouve un logement (16), qui reçoit une extrémité libre (9) d'une seringue (11) ou analogue, pour injecter ou prélever du liquide, logement dont la paroi concentrique au tube (4) forme, avec la surface extérieure du tube (4), un espace annulaire (8) qui est suffisamment important pour recevoir l'extrémité libre (9) de la seringue (11) ou analogue ; et, lors de la mise en place de l'extrémité libre (9) de la seringue (11) ou analogue dans le logement (15), l'extrémité libre de la pièce en élastomère se déplace par rapport au tube (4) de telle sorte qu'elle soit percée par l'extrémité libre (6) du tube (4) et de ce fait réalise une relation d'écoulement liquide entre la seringue (11) ou analogue et le canal (1), caractérisé en ce que la pièce en élastomère (5) est, dans sa zone radialement extérieure (15) et dans la zone de l'extrémité libre (6) du tube (4), comprimée par une douille de serrage (14), sous une pression agissant radialement vers le milieu.

2. Dispositif selon la revendication 1, caractérisé en ce que la pièce en élastomère entoure comme un gobelet le tube (4) jusqu'au voisinage de sa zone de fixation (12), et que la pièce en élastomère (5) est, par son extrémité opposée à l'extrémité libre, fixée à l'extrémité intérieure (13) du cylindre de guidage (3).

3. Dispositif selon la revendication 1, caractérisé en ce que le logement (16) est formé au niveau de l'extrémité libre extérieure (7) du cylindre de guidage (3), et que le logement (16) est formé par un cône femelle de Suer (16), dans lequel l'extrémité libre (9), elle aussi ayant la forme d'un cône mâle de Luer, assure une prise étanche lors de la mise en place de la seringue (11) ou analogues.

4. Dispositif selon l'une des revendications 1 ou 2, caractérisé en ce que la pièce en élastomère (5) présente dans la zone centrale (17) du tube (4) des parois (18) plus minces que dans la zone de l'extrémité libre (6) du tube (4), ce qui forme au moins un espace annulaire libre (19, 20) entre le tube (4) et le cylindre de guidage (3).

5. Dispositif selon l'une des revendications 1 à 4, caractérisé en ce que la douille de serrage présente, au niveau de l'extrémité intérieure, une collerette (21), débordant latéralement vers l'extérieur, destinée à assurer un appui, d'une part contre un épaulement correspondant (22) de la pièce en élastomère (5), d'autre part contre un épaulement (23) du cylindre de guidage (3).

6. Dispositif selon les revendications 3 et 5, caractérisé en ce que l'épaulement (23) du cylindre de guidage (3) est l'extrémité intérieure du cône de Luer femelle (16) du cylindre de guidage (3).

7. Dispositif selon l'une des revendications 1, 2 ou 4, caractérisé en ce que le tube (4) est en une matière plastique et est formé d'une manière monobloc avec l'enveloppe de canal (2).

8. Dispositif selon la revendication 1, caractérisé en ce que le cylindre de guidage (3) peut, en son extrémité extérieure libre, être obturé par un capuchon articulé de recouvrement (24) et, au niveau de son extrémité de fixation intérieur, est, à l'aide d'un dispositif à déclic (25), rapporté à l'enveloppe de canal (2) sous l'effet de la compression axiale d'une collerette radiale (26) de la pièce en élastomère (5).

9. Dispositif selon l'une des revendications 1, 2, 4 ou 5, caractérisé en ce que la pièce en élastomère (5) est en caoutchouc, en latex ou en un mélange de latex et d'une silicone.

Fig. 1

Fig. 2

Fig. 3